# FASCICULE DE BREVET EUROPEEN

(11) **EP 0 506 534 B1**
(45) Date de publication et mention de la délivrance du brevet: **07.02.1996**
(21) Numéro de dépôt: 92400774.3
(22) Date de dépôt: 23.03.1992
(51) Int. Cl.: G01N 1/10

(54) **Tête de prélévement d'échantillons liquides**
Entnahmekopf für Flüssigkeitsproben
Liquid sampling head

(30) Priorité: 25.03.1991 FR 9103574
(43) Date de publication de la demande: 30.09.1992
(73) Titulaire: COGEMA COMPAGNIE GENERALE DES MATIERES NUCLEAIRES, F-78141 Velizy-Villacoublay (FR)
(72) Inventeur: Allain, Jean-Guy, F-50120 Equeurdreville (FR); Kermorgant, Jean-Louis, F-50120 Equeurdreville (FR); Rivalain, Fernand, F-50470 La Glacerie (FR)
(74) Mandataire: Poulin, Gérard

(56) Documents cités:
- EP-A- 0 078 211
- EP-A- 0 296 917
- FR-A- 2 058 751

## Description

L'invention concerne une tête de prélèvement comportant une aiguille dont l'extrémité inférieure plonge dans un liquide à prélever et dont l'extrémité supérieure est apte à transpercer un opercule fermant un cruchon de prélèvement sous vide, de telle sorte qu'une quantité déterminée de liquide soit automatiquement prélevée dans le cruchon sous l'effet d'aspiration provoqué par le vide régnant à l'intérieur du cruchon.

Dans certaines industries telles que l'industrie nucléaire et l'industrie chimique, il peut être nécessaire de procéder à des analyses ponctuelles des liquides présents dans les installations. Cela s'applique notamment à certains liquides radioactifs, dans les installations de traitement de combustibles nucléaires.

Afin de ne pas perturber le fonctionnement de l'installation, on prélève ponctuellement les quantités nécessaires à ces analyses. Comme le décrivent les documents FR-A-1 401 298 et FR-A-2 058 751, une solution connue consiste à utiliser à cet effet un banc de prise d'échantillons liquides comportant plusieurs têtes de prélèvement reliées individuellement à des parties différentes de l'installation, de façon à permettre le prélèvement d'échantillons de liquides radioactifs différents. Le banc de prise d'échantillons comporte en outre un outillage implanté dans une boîte à gants, qui permet de piquer un opercule obturant l'extrémité d'un cruchon sous vide sur l'aiguille de l'une des têtes de prélèvement.Ces dernières sont alors agencées par exemple selon un cercle dans le fond de la boîte à gants, afin qu'un outil monté sur un bouchon tournant disposé coaxialement à ce cercle permette d'accéder indifféremment à chacune des têtes de prélèvement.

Dans un banc ainsi réalisé, chaque tête de prélèvement comprend un pot de prélèvement fixé dans le fond de la boîte à gants et un corps d'aiguille démontable, supportant l'aiguille, qui est emboîté dans un réceptacle du pot de prélèvement. En dessous de ce réceptacle, l'aiguille plonge dans une capacité formée dans le pot de prélèvement et à l'extrémité supérieure de laquelle débouchent une conduite d'entrée et une conduite de sortie d'un fluide à prélever. Ces deux conduites relient la tête de prélèvement à l'installation dans laquelle le fluide doit être prélevé, de telle sorte que ce fluide circule dans la tête de prélèvement au moins lorsqu'un prélèvement doit être effectué sur cette tête. De plus, un passage de faible section relie ce fond de la capacité à l'une des conduites, pour permettre la vidange de la capacité lorsque la circulation du liquide à prélever dans la tête de prélèvement est stoppée.

Dans une tête de prélèvement ainsi conçue, le passage de vidange qui débouche dans le fond de la capacité contribue à créer des turbulences à l'intérieur de celle-ci. Par conséquent, au lieu de ne prélever que du liquide, il arrive fréquemment que les cruchons aspirent une partie importante de gaz, de sorte que la quantité de liquide prélevée dans le cruchon s'avère insuffisante pour les analyses à effectuer.

Cependant, l'existence du passage de vidange ne peut en aucun cas être remise en cause, car la nécessité de vidanger de temps en temps la capacité est impérative pour la qualité des prélèvements effectués.

La présente invention a précisément pour objet une tête de prélèvement d'échantillons liquides comportant un corps d'aiguille dont la forme modifiée permet d'assurer en permanence le remplissage de la capacité en liquide et de tranquiliser au mieux la partie du liquide dans laquelle s'effectue le prélèvement, sans pour autant supprimer le passage de vidange.

Conformément à l'invention, ce résultat est obtenu au moyen d'une tête de prélèvement d'échantillons liquides comprenant un pot de prélèvement pourvu d'un réceptacle dans lequel est reçu un corps d'aiguille démontable portant une aiguille de prélèvement dont une extrémité inférieure plonge dans une capacité formée dans le pot de prélèvement, en dessous du réceptacle, le pot de prélèvement comportant de plus une conduite d'entrée de liquide et une conduite de sortie de liquide débouchant à l'extrémité supérieure de la capacité, et un passage de vidange reliant le fond de la capacité à l'une desdites conduites, caractérisée par le fait que le corps d'aiguille comporte un prolongement entourant l'aiguille et dont une extrémité inférieure obture le passage de vidange lorsque le corps d'aiguille est reçu dans le réceptacle, des trous étant formés dans le prolongement pour faire communiquer la capacité avec un espace délimité autour de l'aiguille, à l'intérieur du prolongement.

Dans une tête de prélèvement ainsi réalisée, l'obturation du passage de vidange par l'extrémité du prolongement du corps d'aiguille assure en permanence le remplissage de la capacité en liquide à prélever. De plus, étant donné que le liquide prélevé se trouve situé dans l'espace formé entre l'aiguille et le prolongement du corps d'aiguille, qui communique avec la capacité par des trous formés dans le prolongement, l'échantillon liquide dans lequel s'effectue le prélèvement est parfaitement tranquilisé. En conséquence, lorsqu'un cruchon est piqué sur l'aiguille, une quantité suffisante de liquide est aspirée dans le cruchon par le vide qui y règne initialement.

Dans un mode de réalisation préféré de l'invention, l'extrémité inférieure du prolongement du corps d'aiguille comporte une partie de diamètre réduit qui pénètre normalement dans une extrémité supérieure de même diamètre du passage de vidange, lorsque le corps d'aiguille est reçu dans le réceptacle.

Par ailleurs, les trous formés dans le prolongement du corps d'aiguille comprennent avantageusement au moins un trou formé sensiblement en face de la conduite d'entrée de liquide et un trou formé sensiblement en face d'une extrémité inférieure de l'aiguille.

On décrira à présent, à titre d'exemple non limitatif, un mode de réalisation préféré de l'invention, en se référant aux dessins annexés, dans lesquels :
- la figure 1 est une vue en coupe longitudinale représentant de façon schématique une tête de prélèvement d'échantillons liquides réalisée conformément à l'invention ; et
- la figure 2 est une vue en coupe longitudinale illustrant à plus grande échelle la partie amovible de la tête de prélèvement de la figure 1.

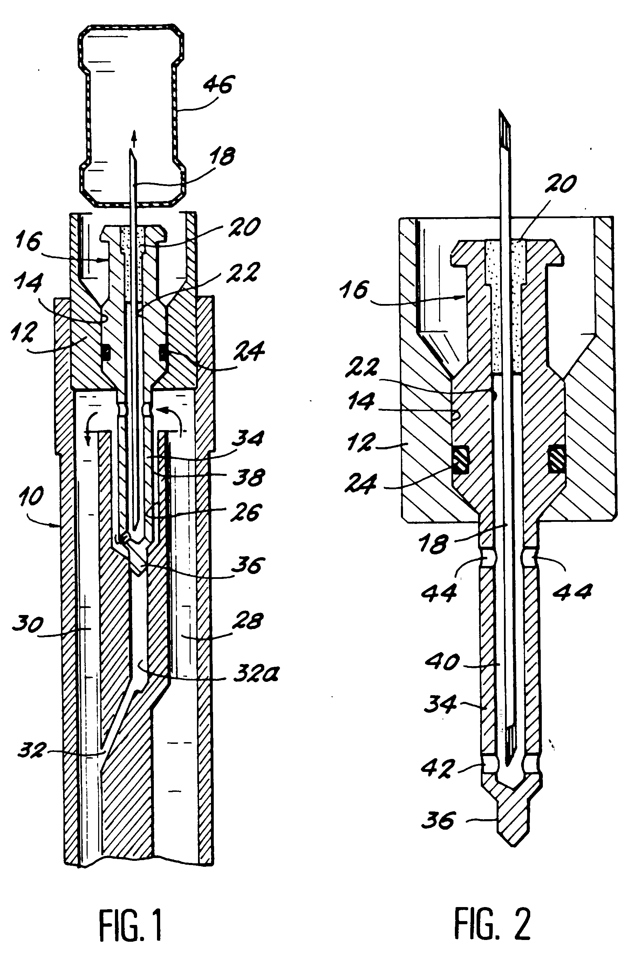

La figure 1 représente une tête de prélèvement d'échantillons liquides prévue pour être utilisée sur un banc de prise d'échantillons tel que celui qui est décrit dans le document FR-A-1 401 298. Cette tête de prélèvement comporte un pot de prélèvement, désigné de façon générale par la référence 10, conçu pour être fixé dans le fond d'une boîte à gants du banc de prise d'échantillons.

Dans sa partie supérieure, le pot de prélèvement 10 comporte un élément 12, en forme de manchon, délimitant intérieurement un réceptacle 14. Un corps d'aiguille démontable, désigné de façon générale par la référence 16, est emboîté à force dans le réceptacle 14, de façon à pouvoir être démonté afin d'être remplacé lorsque cela est nécessaire.

Le corps d'aiguille 16, de forme générale tubulaire, supporte intérieurement une aiguille de prélèvement rectiligne 18. De façon plus précise, le montage de l'aiguille de prélèvement 18 dans le corps d'aiguille 16 est assuré par une pièce de sertissage tubulaire 20 que traverse l'aiguille 18 et qui est montée dans la partie supérieure en gradins d'un alésage 22 formé sur la majeure partie de la hauteur du corps d'aiguille 16.

L'étanchéité entre l'élément 12 et le corps d'aiguille 16 est assurée par un joint d'étanchéité annulaire 24 monté dans une gorge annulaire formée dans la partie du corps d'aiguille 16 reçue dans le réceptacle 14.

En dessous de l'élément 12, le pot de prélèvement 10 forme une capacité 26, agencée selon l'axe du réceptacle 14, et à l'extrémité supérieure de laquelle débouchent une conduite 28 d'entrée du liquide à prélever et une conduite 30 de sortie de ce liquide. En outre, un passage de vidange 32 relie le fond de la capacité 26 à la conduite 30 de sortie du liquide. L'extrémité supérieure 32a de ce passage 32, qui débouche dans la capacité 26, est disposée coaxialement à cette dernière et présente un diamètre uniforme.

Conformément à l'invention et comme l'illustre plus en détail la figure 2, le corps d'aiguille 16 comporte, dans sa partie située en dessous de l'élément 12, un prolongement tubulaire 34, terminé à son extrémité inférieure par une partie cylindrique 36, de diamètre réduit. Cette partie cylindrique 26 pénètre dans l'extrémité supérieure 32a du passage de vidange 32 lorsque le porte-aiguille 16 est reçu dans le réceptacle 14, comme l'illustrent les figures. Le diamètre extérieur de la partie cylindrique 36 est sensiblement égal au diamètre de l'extrémité supérieure 32a du passage 32, de telle sorte que la communication entre la capacité 26 et ce passage est alors interrompue.

Comme l'illustre la figure 1, le diamètre extérieur du prolongement tubulaire 34 du corps d'aiguille 16 est sensiblement inférieur au diamètre intérieur de la capacité 26, de telle sorte qu'un espace annulaire 38, normalement rempli de liquide, est formé dans la capacité 26, autour du prolongement 34.

Par ailleurs, l'alésage 22 formé dans le corps d'aiguille 16 se prolonge vers le bas à l'intérieur du prolongement 34 jusqu'à proximité de la partie cylindrique 36, légèrement au-delà de l'extrémité inférieure de l'aiguille 18, de façon à définir autour de celle-ci un espace annulaire 40.

Cet espace annulaire 40 communique avec l'extérieur par des trous 42 formés dans le prolongement 34 approximativement au niveau de l'extrémité inférieure de l'aiguille 18 et par des trous 44 formés dans le prolongement 34 juste en dessous de l'élément 12, c'est-à-dire en face des extrémités des conduites 28 et 30 débouchant dans la capacité 26.

Dans l'agencement qui vient d'être décrit en se référant aux figures 1 et 2, lorsque le corps d'aiguille 16 est en place dans le réceptacle 14, la partie cylindrique 36 obture l'extrémité supérieure 32a du passage de vidange 32, de sorte que l'espace annulaire 38 formé dans la capacité 26 autour du prolongement 34 est en permanence rempli de liquide. De plus, étant donné que l'espace annulaire 40 formé autour de l'aiguille 18 à l'intérieur du prolongement 34 communique en permanence avec cet espace 38 par les trous 42 et avec les conduites 28 et 30 par les trous 44, la partie de l'espace 40 dans laquelle plonge l'aiguille 18 est également remplie en permanence de liquide dans un état non perturbé. Un renouvellement de ce liquide est cependant assuré en permanence par les trous 42 et 44, ce qui assure une bonne représentativité du liquide prélevé.

Ces différentes caractéristiques permettent donc, lorsque l'opercule d'un cruchon 46 est piqué sur l'extrémité supérieure de l'aiguille 18 comme l'illustre la figure 1, de prélever une quantité de liquide suffisante pour réaliser les analyses désirées. En outre, la qualité de ces analyses n'est pas affectée par une éventuelle stagnation de la partie du liquide dans laquelle s'effectue le prélèvement.

Cependant, la tête de prélèvement selon l'invention permet d'assurer de temps à autre la vidange de la capacité 26 en soulevant le corps d'aiguille 16 ou en le démontant complétement à l'aide d'outils appropriés placés dans la boîte à gants surplombant la tête de prélèvement, ces outils pouvant par exemple être réalisés de la manière décrite dans le document FR-A-2 058 751.

Bien entendu, l'invention n'est pas limitée au mode de réalisation qui vient d'être décrit à titre d'exemple, mais en couvre toutes les variantes. Ainsi, les trous 42 et 44 pourraient être remplacés par des fentes s'étendant au travers du prolongement 34 depuis l'extrémité inférieure de l'aiguille 18 jusqu'à proximité de l'élément 12, dans la zone dans laquelle débouchent les conduites 28 et 30.

## Revendications

1. Tête de prélèvement d'échantillons liquides comprenant un pot de prélèvement (10) pourvu d'un réceptacle (14) dans lequel est reçu un corps d'aiguille démontable (16) portant une aiguille de prélèvement (18) dont une extrémité inférieure plonge dans une capacité (26) formée dans le pot de prélèvement (10), en dessous du réceptacle (14), le pot de prélèvement (10) comportant de plus une conduite d'entrée de liquide (28) et une conduite de sortie de liquide (30) débouchant à l'extrémité supérieure de la capacité (26), et un passage (32) de vidange reliant le fond de la capacité (26) à l'une desdites conduites (28,30), caractérisée par le fait que le corps d'aiguille (16) comporte un prolongement (34) entourant l'aiguille (18) et dont une extrémité inférieure obture le passage de vidange (32) lorsque le corps d'aiguille (16) est reçu dans le réceptacle (14), des trous (42, 44) étant formés dans le prolongement pour faire communiquer la capacité (26) avec un espace (38) délimité autour de l'aiguille (18), à l'intérieur du prolongement(34).

2. Tête de prélèvement selon la revendication 1, caractérisée par le fait que l'extrémité inférieure du prolongement (34) comporte une partie de diamètre réduit (36) qui pénètre normalement dans une extrémité supérieure (32a) de même diamètre dudit passage (32), lorsque le corps d'aiguille (16) est reçu dans le réceptacle.

3. Tête de prélèvement selon l'une quelconque des revendications 1 et 2, caractérisée par le fait que les trous (42,44) comprennent au moins un trou (44) formé sensiblement en face de la conduite d'entrée de liquide (28) et un trou (42) formé sensiblement en face d'une extrémité inférieure de l'aiguille (18).

## Patentansprüche

1. Entnahmekopf für Flüssigkeitsproben, der einen Entnahmetopf (10) enthält, welcher mit einem Sammelbehälter (14) ausgestattet ist, der einen demontierbaren Nadelkörper (16) aufnimmt, welcher eine Entnahmenadel (18) trägt, deren unteres Ende in einen Innenraum (26) taucht, der in dem Entnahmetopf (10) unter dem Sammelbehälter (14) gebildet wird, wobei der Entnahmetopf (10) weiterhin eine Flüssigkeitszuführleitung (28) und eine Flüssigkeitabführleitung (30) enthält, die in das obere Ende des Innenraums (26) münden, und einen Entleerungsdurchgang (32), der den Boden des Innenraums (26) mit einer der genannten Leitungen (28, 30) verbindet, dadurch gekennzeichnet, daß der Nadelkörper (16) eine Verlängerung (34) besitzt, die die Nadel (18) umgibt und deren unteres Ende den Entleerungsdurchgang (32) versperrt, während der Nadelkörper (16) in dem Sammelbehälter (14) aufgenommen wird, wobei in der Verlängerung Löcher (42, 44) eingearbeitet sind, um den Innenraum (26) mit einem Raum (38) in Verbindung zu bringen, der um die Nadel (18) herum im Inneren der Verlängerung (34) abgegrenzt wird.

2. Entnahmekopf nach Anspruch 1, dadurch gekennzeichnet, daß das untere Ende der Verlängerung (34) einen Abschnitt mit verringertem Durchmesser (36) besitzt, der normalerweise in das obere Ende (32a) des genannten Durchgangs (32) eindringt, das denselben Durchmesser hat, wenn der Nadelkörper (16) in dem Sammelbehälter aufgenommen ist.

3. Entnahmekopf nach einem der Ansprüche 1 und 2, dadurch gekennzeichnet, daß sich unter den Löchern (42, 44) mindestens ein Loch (44) befindet, daß sich fast gegenüber der Flüssigkeitszuführleitung (28) befindet, und ein Loch (42), daß sich fast gegenüber dem unteren Ende der Nadel (18) befindet.

## Claims

1. Head for taking liquid samples comprising a sampling tank (10) having a receptacle (14) in which is received a dismantlable needle body (16) carrying a sampling needle (18), whereof a lower end is immersed in a cavity (26) formed in the sampling tank (10), below the receptacle (14), the sampling tank (10) also having a liquid intake tube (28) and a liquid discharge tube (30) issuing at the upper end of the cavity (26) and a draining passage (32) connecting the bottom of the cavity (26) to one of the said tubes (28, 30), characterized in that the needle body (16) has an extension (34) surrounding the needle (18) and whereof a lower end seals the draining passage (32) when said needle body (16) is received in the receptacle (14), holes (42, 44) being formed in the extension in order to link the cavity (26) with a space (38) defined around the needle (18) within the extension (34).

2. Sampling head according to claim 1, characterized in that the lower end of the extension (34) has a reduced diameter portion (36), which normally enters an upper end (32a) having the same diameter as said passage (32), when the needle body (16) is received in the receptacle.

3. Sampling head according to either of the claims 1 and 2, characterized in that the holes (42, 44) have at least one hole (44) formed substantially facing the liquid intake tube (28) and one hole (42) formed substantially facing the lower end of the needle (18).
